# EUROPÄISCHE PATENTSCHRIFT

(11) **EP 1 203 565 B1**
(45) Veröffentlichungstag und Bekanntmachung des Hinweises auf die Patenterteilung: **10.10.2007**
(21) Anmeldenummer: 01125715.1
(22) Anmeldetag: 27.10.2001
(51) Int. Cl.: A61B 10/00

(54) **Endoskopischer Probenehmer für insbesondere Knorpelmaterial**
Endoscopic biopsy device, in particular for cartilage
Dispositif de biopsie endoscopique, notamment pour cartilage

(30) Priorität: 02.11.2000 DE 10054265
(43) Veröffentlichungstag der Anmeldung: 08.05.2002
(73) Patentinhaber: Richard Wolf GmbH, 75438 Knittlingen (DE)
(72) Erfinder: Freier, Mark, 75038 Oberderdingen (DE); Heckele, Helmut, 75438 Knittlingen (DE); Körner, Eberhard, Dipl.-Ing. (FH), 75015 Bretten (DE)
(74) Vertreter: Hemmer, Arnd

(56) Entgegenhaltungen:
- WO-A-97/11646
- DE-A- 1 855 179
- FR-A- 2 450 597
- US-A- 4 651 752
- US-A- 4 800 896
- US-A- 4 926 877
- US-A- 5 683 406
- US-A- 6 030 400

## Beschreibung

Die Erfindung geht aus von einem endoskopischen Probenehmer für insbesondere Knorpelmaterial, gemäß dem Oberbegriff des Patentanspruchs 1.

Instrumente zum Abschaben von Knorpel- und Knochenmaterial sind in der Medizintechnik bekannt. So beschreiben WO 97/11646 A und US 5,683,406 A solche Instrumente für den Bereich der Zahnmedizin bzw. Kieferchirurgie. Die dort offenbarten Instrumente weisen einen länglichen, rinnenförmigen gewölbten Grundkörper auf. Das distale Ende dieses Grundkörpers wird durch eine dort verschiebbar angeordnete Abdeckung verschlossen. Diese Abdeckung ist distalseitig derart geschlitzt, dass sie eine hobelartige Schneidkante zum Abschaben von Knochenmaterial bildet. Zum Abschaben von Knorpel- oder Knochenmaterial ist bei diesen Instrumenten eine zu der Längsachse des Instrumentes abgewinkelte Bewegungsführung erforderlich.

Ein endoskopischer Probenehmer ist in dem DE-Gebrauchsmuster 1 855 179 beschrieben. Er besteht aus einem Hohlschaft mit einer darin axial verstellbaren Betätigungsstange, einer an dem distalen Ende des Hohlschaftes verschwenkbar angeordneten Laffe und einer am proximalen Ende des Hohlschaftes angeordneten, scherenartigen Handhabe. Diese Handhabe weist ein unbewegliches Griffteil, das an dem Hohlschaft starr befestigt ist, und ein bewegliches Griffteil, das mit dem unbeweglichen Griffteil verschwenkbar verbunden ist und an dem proximalen Ende der Betätigungsstange angreift, auf. Durch Betätigen des beweglichen Griffteiles wird die Laffe mittels der Betätigungsstange in Bezug auf den Hohlschaft verschwenkt, so dass durch Vor- und Zurückschwenken der Laffe Knorpelgewebe, insbesondere von menschlichen Gelenken, durch Abschaben gelöst werden kann. Die abgeschabten Knorpelteilchen werden durch Herausspülen aus dem Körper entfernt und sind aus der Spülflüssigkeit nur sehr umständlich und schwierig zu gewinnen, um daraus neue und reimplantierbare Knorpelmasse zu züchten. Ferner hat es sich als unzulänglich erwiesen, die gewünschten Knorpelteilchen nur mit der Laffe allein aus dem Gelenk oder einem anderen erkrankten Knorpelbereich eines Patienten zu entnehmen, weil die abgeschabten Knorpelteilchen beim Herausziehen des Probenehmers aus der betreffenden Körperhöhle zum größten Teil wieder verlorengehen. Ein weiterer

Nachteil dieses bekannten Probenehmers besteht darin, dass die Laffe zum Abschabevorgang erheblich quergestellt werden muss und somit aufgrund ihrer Bauweise und zur Ausübung ihrer Funktion beträchtlichen Platz in der Körperhöhle des Gelenkes oder dergleichen benötigt.

Des weiteren sind Biopsiezangen mit einem oder zwei verschwenkbaren, hohlen Backenteilen zum sicheren Entnehmen von Gewebeproben bekannt. Die Backenteile befinden sich am distalen Ende eines Hohlschaftes, an dessen proximalem Ende eine Handhabe zur Betätigung der Backenteile vorgesehen ist. Diese Zangen eignen sich im wesentlichen nur zum Entnehmen von Weichgewebe und nicht für Knorpelgewebe oder dergleichen, da dieses beträchtlich härter ist. Im Übrigen benötigen auch diese Backenteile zur Ausübung ihrer Funktion relativ viel Platz, weil sie quer zur Längsrichtung des Hohlschaftes aufgespreizt werden müssen.

Die Aufgabe der Erfindung besteht in der Verbessserung eines endoskopischen Probenehmers der einleitend angeführten Art, der bei geringem distalen Platzbedarf eine sichere und schnelle Entnahme von Knorpelmaterial aus insbesondere menschlichen Körperhöhlen gewährleistet.

Die Lösung dieser Aufgabe ist in dem Anspruch 1 angegeben.

Mit dieser Lösung kann Knorpelmaterial sicher und schnell von einer Knorpelstelle, z. B. von einem Kniegelenk eines Patienten, unverlierbar entnommen werden, um zur Gewinnung bzw. Züchtung von neuem Knorpelzellmaterial verwendet werden zu können, welches dann an einer beschädigten Knorpelstelle im Körper des Patienten reimplantiert wird. Nachdem gesundes Knorpelmaterial von der gewünschten Knorpelstelle des Patienten durch Schaben in die Mulde der Laffe gelangt ist, wird die Mulde durch Vorschieben der erfindungsgemäßen Abdeckung geschlossen, so dass das abgetrennte Knorpelmaterial beim Herausziehen des Probenehmers aus dem Körper des Patienten nicht verlorengehen kann. Ein weiterer Vorteil des erfindungsgemäßen Probenehmers besteht darin, dass die Abdeckung für die Mulde der Laffe äußerst platzsparend am distalen Ende des Probenehmers angeordnet ist und dadurch praktisch sehr wenig Raum beansprucht, zumal sie sich in Nichtarbeitsstellung in zurückgezogener Position in dem Hohlschaft befindet. Weiterhin ist vorteilhaft, dass die Abdeckung einfach und mit sehr geringen Kosten herzustellen ist.

In einer vorteilhaften Ausgestaltung des erfindungsgemäßen Probenehmers besteht die Abdeckung für die Mulde der Laffe aus einer Metallzunge. In dem Falle, dass die Laffe in Bezug auf den Hohlschaft in einem gewissen Winkel retrograd abgewinkelt ist, besteht die Abdeckung aus einem flexiblen Material. Hierbei ist es vorteilhaft, dass in dem Hohlschaft ein axial beweglicher Niederhalter vorgesehen ist, um die Abdeckung auf der Laffe in Schließlage zu halten.

Die Erfindung ist nachstehend anhand eines in den anliegenden Zeichnungen dargestellten Ausführungsbeispieles näher erläutert. Es zeigen:
- Figur 1: eine Seitenansicht auf den Probenehmer,
- Figur 2: einen Axialschnitt durch den distalen Endbereich des Probenehmers,
- Figur 3: einen teilweisen Axialschnitt durch den Probenehmer nach Fig.1.

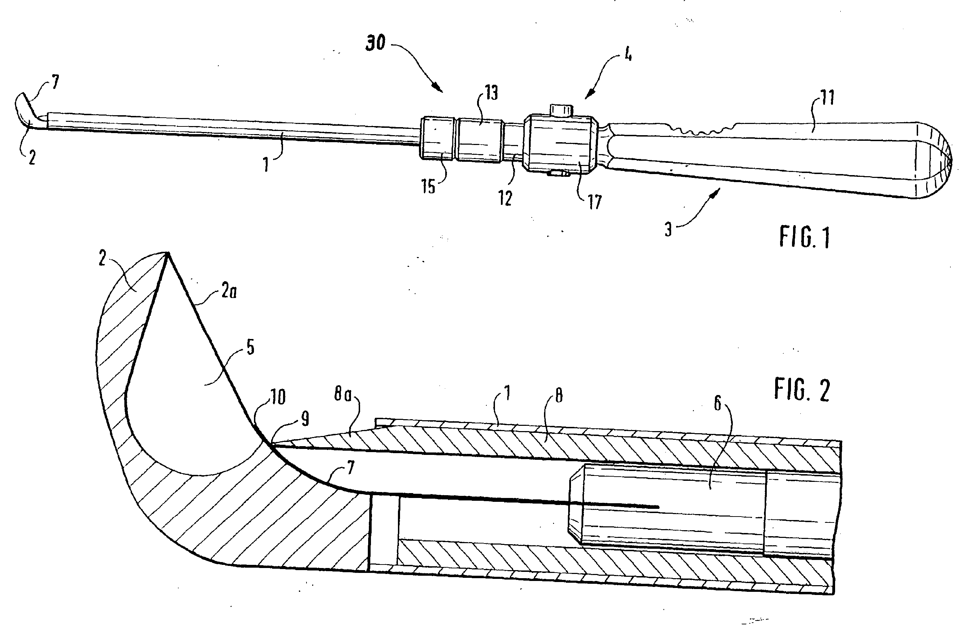

Der in Figur 1 allgemein mit 30 bezeichnete Probenehmer umfasst einen Hohlschaft 1 mit einer Laffe 2, die starr und unlösbar an dem distalen Ende des Hohlschaftes befestigt ist, und eine Handhabungseinrichtung 3 mit einem Betätigungsmechanismus 4. Die Laffe 2 weist eine löffelartige oder schalenartige Mulde 5 auf, in welche Knorpelmaterial, Knochenmaterial oder ähnlich hartes Material aus einer Körperhöhle eines Lebewesens, vorzugsweise eines Patienten, gelangt, welches Material durch Abschaben gewonnen wird. Hierzu besitzt die Laffe einen scharfen Rand 2a. In dem Hohlschaft 1 befindet sich eine Betätigungsstange 6, an deren distalem Ende eine zungenförmige Abdeckung 7 für die Mulde 5 der Laffe 2 (Fig. 2) befestigt ist. Das Material für die Abdeckung 7 ist vorzugsweise dünnes Metall. Die Abdeckung kann streifenförmig ausgebildet sein und in ihrem Abdeckungsabschnitt eine Umfangsform aufweisen, die der oder im wesentlichen der Umfangsform der Mulde 5 entspricht. Wesentlich hierbei ist, dass die Mulde 5 durch die Abdeckung 7 ausreichend abgedeckt wird, damit das gewonnene Knorpelmaterial beim Herausziehen des Probenehmers 30 aus der Körperhöhle des Patienten nicht verlorengeht.

Die Laffe 2 kann sich koaxial zu dem Hohlschaft 1 erstrecken. Es ist jedoch auch möglich, die Laffe in Bezug auf den Hohlschaft 1 in einem gewissen Winkel verlaufend vorzusehen, wie es die Figuren 1, 2 und 3 zeigen. In diesem Fall besteht die Adeckung 7 aus einem biegeelastischen Material, so dass sie sich selbsttätig biegt und sich dem schrägen Verlauf des Randes 3 der Laffe 2 anpasst, wenn die Betätigungsstange 6 vorgeschoben wird. Um eine sichere Führung der Abdeckung 7 an der Laffe 2 zu gewährleisten, ist es vorteilhaft, einen Niederhalter 8 vorzusehen, der einen distalen Fortsatz 8 a aufweist, der zur Laffe 2 einen Spalt 9 zur Führung der Abdeckung 7 bestimmt. Der Niederhalter 8 kann aus einem im Schaft 1 unlösbar festgelegten Zylinderabschnitt bestehen oder einen Teil des Schafts 1 bilden. Die Betätigungsstange 6 mit der Abdeckung 7 wird zu ihrer axialen Verschiebung durch den Niederhalter geführt. Mit dem Fortsatz 8 a des Niederhalters 8 wird auch erreicht, dass die über die Mulde 5 geschobene Abdeckung 7 auf der Laffe 2 in Schließlage gehalten wird.

In weiterer vorteilhafter Ausgestaltung kann der vordere Rand 10 der Abdeckung 7 als Schneide 10 ausgebildet sein, um ein Abtrennen von Knorpelgewebe oder dergleichen zu erleichtern.

Zur axialen Betätigung der Betätigungsstange 6 mit der Abdeckung 7 ist eine geeignete Handhabungseinrichtung vorgesehen. Diese Einrichtung ist nicht Gegenstand der vorliegenden Erfindung und daher nur kurz beschrieben.

Der Hohlschaft 1 mit der Laffe 2 und die Betätigungsstange 6 mit der Abdeckung 7 bilden eine zerlegbare Baueinheit, die mit der Handhabungseinrichtung 3 lösbar verbunden ist. Diese Einrichtung weist einen Handgriff 11 mit einem distalen Hülsenabschnitt 12 als Bauteil des Betätigungsmechanismus 4 auf. Auf den Hülsenabschnitt 12 ist eine Schraubhülse 13 aufschraubbar, um das proximale Endteil 14 des Niederhalters 8 in dem Hülsenabschnitt 12 zu befestigen. Des weiteren ist eine Überwurfmutter 15 auf das proximale Ende des Endbauteils 14 aufschraubbar, um den Hohlschaft 1 an der Einrichtung 3 zu befestigen.

Der Mechanismus 4 umfasst weiter einen auf dem Hülsenabschnitt 12 axial verschiebbar angeordneten, äußeren zylindrischen Ring 17 und einen innerhalb des Hülsenabschnittes 12 auf der Betätigungsstange 6 starr befestigten, inneren zylindrischen Ring 18 sowie einen Feststellriegel 19. Der Riegel 19 durchquert die Ringe 17 und 18 sowie ein axiales Langloch 20 des Hülsenabschnittes 12. Ferner ist der Riegel 19 gemäß dem Doppelpfeil 21 verstellbar.

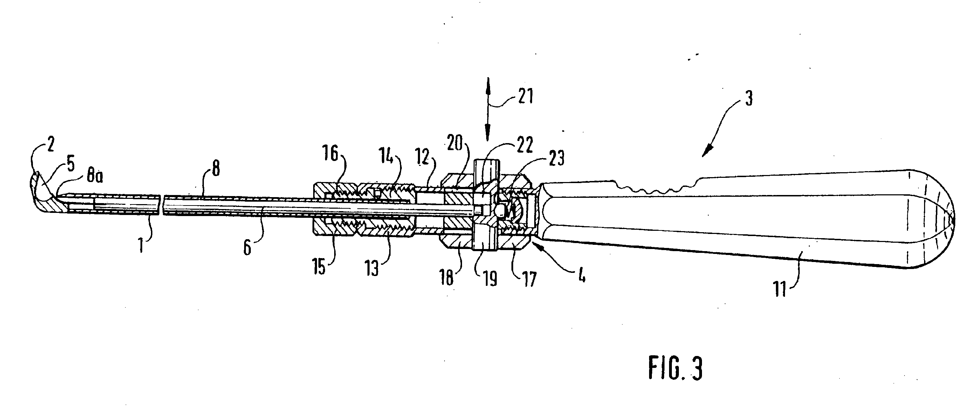

Der Riegel 19 besitzt außerdem eine geeignete Aufnahme 22 für das formschlüssige Einsetzen des proximalen Endes der Betätigungsstange 6. Ferner ist in dem inneren zylindrischen Ring 18 des Mechanismus 4 eine Kugelrastausbildung 23 vorgesehen, um den Riegel 19 in einer Verriegelungsstellung und in einer Lösestellung festzulegen. In Figur 3 ist die Kugelrasteinrichtung 23 in ihrer Verriegelungsstellung gezeigt.

Zur axialen Betätigung der Abdeckung 7 wird der äußere Ring 17 in Axialrichtung, d. h. entsprechend dem Verlauf des Hohlschaftes 1, betätigt. Somit wird auch die Betätigungsstange 6 axial bewegt, so dass die Abdeckung 7 über die Mulde 5 der Laffe 2 geschoben werden kann. Um die Mulde 5 wieder zu öffnen, wird der äußere Ring 17 wieder zurückgeschoben, d. h. proximalwärts bewegt.

## Patentansprüche

1. Endoskopischer Probenehmer (30) für insbesondere Knorpelmaterial, umfassend einen Hohlschaft (1) mit einer an seinem distalen Ende angeordneten Laffe (2), welche einen scharfen Rand 2a zum Abschaben von Knorpel- oder Knochenmaterial aufweist, eine am proximalen Ende des Hohlschaftes vorgesehene Handhabungseinrichtung (3) mit einem Betätigungsorgan (4) und eine in dem Hohlschaft axial bewegbare, mit dem Betätigungsorgan verbindbare Betätigungsstange (6), **dadurch gekennzeichnet, dass** der Mulde (5) der starr an dem Hohlschaft (1) angeordneten Laffe (2) eine vor- und zurückverschiebbare Abdeckung (7) zugeordnet ist, die distal an der Betätigungsstange (6) befestigt ist.

2. Probenehmer nach Anspruch 1 **dadurch gekennzeichnet, dass** die Abdeckung (7) für die Mulde (5) der Laffe (2) aus einer Metallzunge besteht.

3. Probenehmer nach Anspruch 1 oder 2, **dadurch gekennzeichnet, dass** die Abdeckung (7) für die Mulde (5) der Laffe (2) für den Bereich der Mulde eine Umfangsform aufweist, die der oder im wesentlichen der Umfangsform der Mulde entspricht.

4. Probenehmer nach Anspruch 1, 2 oder 3, **dadurch gekennzeichnet, dass** in dem Hohlschaft (1) ein Niederhalter (8) zum Führen und Halten der Abdeckung (7) in Schließlage auf der Laffe (2) vorgesehen ist.

5. Probenehmer nach einem der Ansprüche 1 bis 4, **dadurch gekennzeichnet, dass** die Laffe (2) in Bezug auf den Hohlschaft (1) abgewinkelt ist.

6. Probenehmer nach Anspruch 5, **dadurch gekennzeichnet, dass** die Abdeckung (7) für die Mulde (5) der Laffe (2) biegeelastisch ist.

7. Probenehmer nach einem der Ansprüche 1 bis 6, **dadurch gekennzeichnet, dass** die Abdeckung (7) für die Mulde (5) der Laffe (2) eine vordere Schneidkante (10) aufweist.

## Claims

1. An endoscopic sampling or biopsy device (30) in particular for cartilage material, comprising a hollow shank (1) with a scoop (2) arranged at its distal end, which comprises a sharp edge (2a) for scraping away cartilage material or bone material, a handling means (3) with an actuation element (4), said handling means being provided at the proximal end of the hollow shank, and an actuation rod (6) which is axially movable in the hollow shank and may be connected to the actuation element, **characterised in that** a cover (7) which may be pushed forwards and back, and is fastened distally on the actuation rod (6), is allocated to the trough (5) of the scoop (2) arranged rigidly on the hollow shank (1).

2. A biopsy device according to claim 1, **characterised in that** the cover (7) for the trough (5) of the scoop (2) consists of a metal tongue.

3. A biopsy device according to claim 1 or 2, **characterised in that** the cover (7) for the trough (5) of the scoop (2), for the region of the trough, has a peripheral shape which corresponds to, or essentially to the peripheral shape of the trough.

4. A biopsy device according to claim 1, 2 or 3, **characterised in that** a holding-down means (8) for guiding and holding the cover (7) in the closure position on the scoop (2) is provided in the hollow shank (1).

5. A biopsy device according to one of the claims 1 to 4, **characterised in that** the scoop (2) is angled with respect to the hollow shank (1).

6. A biopsy device according to claim 5, **characterised in that** the cover (7) for the trough (5) of the scoop (2) is bending-elastic.

7. A biopsy device according to one of the claims 1 to 6, **characterised in that** the cover (7) for the trough (5) of the scoop (2) has a front cutting edge (10).

## Revendications

1. Dispositif de biopsie endoscopique (30), en particulier pour cartilage, comportant une tige creuse (1) avec une patte (2) disposée à son extrémité distale, qui présente un bord (2a) tranchant pour gratter le cartilage ou l'os, un dispositif de manipulation (3) prévu à l'extrémité proximale de la tige creuse, muni d'un organe de commande (4), et une tige de commande (6) mobile axialement dans la tige creuse et pouvant être assemblée avec l'organe de commande, **caractérisé en ce qu'**un cache de protection (7) pouvant avancer et reculer est associé à la partie creuse (5) de la patte (2) disposée de manière rigide sur la tige creuse (1), ledit cache étant fixé distalement à la tige de commande (6).

2. Dispositif de biopsie selon la revendication 1, **caractérisé en ce que** le cache de protection (7) pour la partie creuse (5) de la patte (2) est constitué d'une languette métallique.

3. Dispositif de biopsie selon la revendication 1 ou 2, **caractérisé en ce que** le cache de protection (7) pour la partie creuse (5) de la patte (2) présente, dans la zone de la partie creuse, une forme périphérique qui correspond ou correspond sensiblement à la forme périphérique de la partie creuse.

4. Dispositif de biopsie selon la revendication 1, 2 ou 3, **caractérisé en ce que** dans la tige creuse (1), il est prévu un serre-flan (8) pour le guidage et le maintien du cache de protection (7) en position de fermeture sur la patte (2).

5. Dispositif de biopsie selon l'une des revendications 1 à 4, **caractérisé en ce que** la patte (2) forme un angle par rapport à la tige creuse (1).

6. Dispositif de biopsie selon la revendication 5, **caractérisé en ce que** le cache de protection (7) pour la partie creuse (5) de la patte (2) est élastique en flexion.

7. Dispositif de biopsie selon l'une des revendications 1 à 6, **caractérisé en ce que** le cache de protection (7) pour la partie creuse (5) de la patte (2) présente une arête de coupe (10) antérieure.
